# EUROPEAN PATENT APPLICATION

(11) **EP 3 680 915 A1**
(43) Date of publication of application: **15.07.2020**
(21) Application number: 19151679.8
(22) Date of filing: 14.01.2019
(51) Int. Cl.: G16H 40/63, G16H 20/70, G09B 19/24

(54) **METHOD, SYSTEM, CLIENT COMPUTING DEVICE, AND SERVER COMPUTING DEVICE FOR ENHANCING THE EFFICIENCY OF THE USE OF DIGITAL EQUIPMENT BY CHANGING HABITS OF A USER OF A CLIENT COMPUTING DEVICE**

(71) Applicant: Digital Attitude S.r.l., 20123 Milano (IT)
(72) Inventor: LUSENTI, Simone, 20123 Milano (IT); ARGENTON, Luca, 20123 Milano (IT); FALCONI, Filippo Muzi, 20123 Milano (IT)
(74) Representative: Schumacher & Willsau

(57) **Abstract**

The invention is related to methods, systems, client computing devices, and server computing devices for enhancing the efficiency of the use of digital equipment by changing habits of a user of a client computing device. The invention encompasses: monitoring, for a first monitoring time period, the behavior of the user interacting with a client user interface of the client computing device and collecting first user behavior data. Transmitting, with a client transceiver of the client computing device, the first user behavior data to a server transceiver of a server computing device. Receiving, with the server transceiver, the first user behavior data. Storing, in a server memory of the server computing device, the first user behavior data. Determining a user habit, based on the first user behavior data. Selecting, by the server computing device, a first user habit change stimulus from a plurality of user habit change stimuli, based on the user habit, the first user behavior data and the behavior data of other users stored in the server memory. Transmitting, with the server transceiver, the first habit change stimulus to the client transceiver. Receiving, with the client transceiver, the first user habit change stimulus. Applying, via the client user interface, the first user habit change stimulus to the user. Monitoring, for a second monitoring time period, the behavior of the user interacting with the client user interface and collecting second user behavior data. Determining whether the user has changed the first user habit. And, finally, if the user has changed the first user habit in the desired direction and if there are further user habits to be changed, concentrating on a further user habit.

## Description

### BACKGROUND OF THE INVENTION

The invention is related to methods, systems, client computing devices, and server computing devices for enhancing the efficiency of the use of digital equipment by changing habits of a user of a client computing device. As regards digital equipment, employees are constantly being asked to adapt to new applications, functions, and workflows that promise to improve organizational productivity, collaboration, and efficiency. Although they may be enthusiastic initially, constant requirements for software updates and deployment can lead to frustration and a new form of stress known as Technostress. The present invention aims to make technology adoption simple. It helps to empower human beings in their interaction with technologies by shaping new and healthy behaviors. To reach this goal, the invention helps the user discover what methods, systems, client computing devices, and server computing devices are necessary to "coach" users to "install" new habits, facilitating technology adoption and finally to reduce Technostress.

The adoption and use of new technological solutions have redefined organizational structures and business processes, changing the means of interaction among and between individuals and organizations. As such, for example, two-thirds of the CEOs of the European Fortune 500 enterprises have Digital Transformation programs at the center of their corporate strategy. Employees must continually adapt to new processes and technologies, which can lead to a complex phenomenon called Technostress, a modern disease of adaptation caused by the inability to cope with new computer technologies in a healthy way. As a multidimensional construct, Technostress has specific consequences repeatedly cited in scientific literature:
- Physical consequences, such as eye strain, headaches, muscle tension, rapid heart rate, increased blood pressure, difficulty breathing.
- Behavioral consequences, like insomnia, uncooperativeness and unwillingness, social withdrawal.
- Psychological consequences, such as fear that computers may replace human roles, professional jealousy of technological competency, demotivation, uncertainty about the job role.
- Emotional consequences, like irritability, loss of temper, anxiety, feelings of indifference, frustration, lack of appreciation, depression, and guilt.

From an organizational perspective, Technostress may result not only in abandoned software and negative return on investment (ROI), as well as wasted time and resources, but also in lower levels of commitment and engagement.

The impact of Technostress within enterprises in many cases must be considered alongside a distinctive demographic trend, for example the progressive ageing of the European workforce. For example the European Union is experiencing an unprecedented demographic transformation marked by an ageing population, higher life expectancy, a declining fertility rate, the growing old-age dependency ratio, and the resulting consequences on the workforce, with a shift in its composition from relatively young to relatively old workers. For example, the share of workers aged 55+ in the workforce is expected to increase substantially over the coming decades.

A strong correlation has emerged between ageing workforce and Technostress, as senior workers are those experiencing the highest level of frustration when dealing with new technology adoption within their organization. The elderly lag behind in using and benefiting from the information and communication technology (ICT), even if ICT usage offers them significant potential for remaining independent for longer, and fulfilling professional duties and responsibilities. They often fail to adapt to new applications or software because they have developed routines and habits that they struggle to change. Therefore, they are not able to keep up with organizational demands, and experience decreased productivity levels, lower levels of job satisfaction, and reduced organizational commitment.

In accordance with the prior art, different tools and approaches are used to boost technology adoption within modern organizations. Online training, tutorials, walk-throughs, as well as customer service and support software, are the most frequently used. These solutions are sporadic, do not influence learning on the job, and are not tailored to specific user needs. Furthermore, these existing solutions are only technology-driven: no attention is given to empowering the quality of employees' experiences when dealing with new technologies within the organization. Consequently, while organizations make significant investments in technologies, employees struggle to adopt them because of technical, motivational, and psychological issues.

To address the strong need for technological adoption and to allow, for example, senior workers and people with low technological literacy to be more productive and effective, the market requires new solutions that are able to guide them in the process of technology adoption.

It is therefore an object of the present invention to provide methods, systems, client computing devices, and server computing devices that simplify the technology adoption by taking care of individual needs, interests, and resistance to change.

### BRIEF DESCRIPTION OF THE SEVERAL VIEWS OF THE DRAWINGS

FIG. 1 is flow chart that illustrates one embodiment of the method in accordance with the invention, and that also illustrates the operation of one embodiment of the system, the client computing device, and the server computing device in accordance with the invention; and
FIG. 2 is block diagram that illustrates one embodiment of the system, the client computing device, and the server computing device in accordance with the invention, wherein the illustrated system is also capable to execute the method of FIG.1.

### DETAILED DESCRIPTION OF THE INVENTION

In accordance with a first embodiment the invention solves the above object by providing a method for enhancing the efficiency of the use of digital equipment by changing habits of a user of a client computing device, the method comprising the following steps: monitoring, for a first monitoring time period, the behavior of the user interacting with a client user interface of the client computing device and collecting first user behavior data; transmitting, with a client transceiver of the client computing device, the first user behavior data to a server transceiver of a server computing device; receiving, with the server transceiver, the first user behavior data; storing, in a server memory of the server computing device, the first user behavior data; determining a user habit, based on the first user behavior data; selecting, by the server computing device, a first user habit change stimulus from a plurality of user habit change stimuli, based on the user habit, the first user behavior data, and behavior data of other users stored in the server memory; transmitting, with the server transceiver, the first habit change stimulus to the client transceiver; receiving, with the client transceiver, the first user habit change stimulus; applying, via the client user interface, the first user habit change stimulus to the user; monitoring, for a second monitoring time period, the behavior of the user interacting with the client user interface and collecting second user behavior data; determining whether the user has changed the user habit, based on the second user behavior data; and, if the user has changed the user habit in the desired direction and if there are further user habits to be changed, concentrating on a further user habit. This solution satisfies the clear market needs, overcomes the current obstacles facing the adoption of new technologies, and combats Technostress. The invention provides a solution that is unique, effective, and easy to implement. At its core, it coaches users to build and maintain new habits, facilitating the adoption of new technologies and reducing Technostress in, for example, as little as 90 days. By exploiting the potential offered by Artificial Intelligence and integrating a deep expertise of psychology and change management, the invention offers to workers personalized, real-time assistance that deeply impacts users' routines and behaviors. Thanks to an effortless, ever-present training and education system, the invention reassures the user thereby increasing his/her levels of patience and perceived self-efficacy. As a responsive and personalized guide, it also reduces employees' anxiety and concerns, guiding them through using and familiarizing with new technology applications, and reassuring them when facing problems. Employees feel involved because they can "experiment" and innovate their computer use, while also interacting with a human-like virtual assistant. The invention provides a way to impact users' behaviors at three core levels:
- Acceptance, leading users to gain a better understanding of the focus technology and to acknowledge inherent personal advantages by providing meaningful information;
- Sustainability, encouraging the use and reinforcement of new behaviors over time, transforming repeated actions into habits that will eventually result in the creation of new abilities;
- Ownership, nurturing the feeling of technological mastery, shedding the feeling of being controlled by technology itself, and reducing Technostress.

In accordance with some embodiments, the method further comprises the following steps: determining, after the first monitoring time period, a user baseline, based on the first user behavior data, the user baseline indicating a set of user habits the user actually has and that affect the efficiency of the use of digital equipment; and transmitting, with the client transceiver, the user baseline to the server transceiver. The first monitoring period may, for example, last ten days, and it may be referred to as a "silent mode" because during this period preferably no stimuli are applied to the user. This does not exclude that the user is pulsed with questions, for example two questions per day, to collect significant user behavior data. During the first monitoring period, the system preferably remains silent and starts to gather user behavior data related to the user's technology usage patterns. It analyses daily routines and behaviors of interaction with the technology to identify blocking habits and set an individual baseline. A user baseline can be determined on both sides, the client side and/or the server side, depending on the embodiment. In the latter case in may be sufficient, that the server computing device receives the first user behavior data to determine a user baseline.

It is preferred that the step of determining a user habit is performed by the server computing device, based on the user baseline. It would also be possible that the client computing device determines the presently most important user habit that should be addressed, but in view of the fact that the server computing device preferably has access to user behavior data from many users, it can make the decision on a broader basis.

The step of selecting a first user habit change stimulus from the plurality of user habit change stimuli preferably includes selecting from a plurality of tip stimuli, prime stimuli, nudge stimuli, and feedback stimuli. To make an optimal choice, the server computing device, which could also be referred to as "brain", for example can be a multi-tenant, cloud-based application, which implements the psychological logic previously described. In such a case it uses tailored rule engines, machine-learning code and sophisticated tools to record and parse the incoming data from the client computing device, and, preferably, from many client computing devices. The server computing device is preferably able to assign meaning to each and every action collected by the client computing devices, and to construct an appropriate and user-tailored response, which is then sent back to the client computing device for the user to visualize and interact with. These elements can be implemented as an Internet of Things architecture, i.e. many devices with reduced computational resources send a raw stream of sensor data to a central unit for further processing and analysis.

A prime stimulus can, for example, be a stimulus that influences the near-term future thoughts and actions of a user, even though they may not seem to be connected. A prime stimulus is preferably applied before a user performs a core action to convey a customized message about technology and its features, highlighting its potential for the user.

A nudge stimulus is preferably a stimulus concerning any aspect of a given choice architecture that alters the behavior of users in a predictable way without removing any options. A nudge stimulus can be applied while the user performs an action or an action is taking place, wherein a nudge stimulus includes, for example, short sentences or graphical tools to persuade the user towards the desired user behavior.

A feedback stimulus is helpful information that is given to the user to indicate what can be done to improve adoption after a core action is performed. The feedback stimulus can be positive feedback, rewarding the action taken, or corrective feedback, signaling the possibility to correct a previous mistake.

The step of selecting a first user habit change stimulus from the plurality of user habit change stimuli preferably includes considering results that have been achieved with other users. As mentioned above, the selection preferably is based on Artificial Intelligence to coach users to build and maintain technology-related habits, facilitating the adoption of focus technologies and reducing Technostress.

The step of determining the user habit preferably includes determining a core user habit that is needed to enhance the efficiency of the use of digital equipment. Advantageously the algorithm provided in accordance with the present invention also "understands" where, when, and how to interact with the user (triggers) to achieve optimal results. The core habit could, for example, be identified from one of these clusters: a) better storage and file organization to create shared value for the company; b) full accessibility from other devices, including tablet or smartphone, to enable smart ways of working; c) increased sharing to empower co-design and co-working practices.

The object of the invention mentioned above is also solved by a system for enhancing the efficiency of the use of digital equipment by changing habits of a user of a client computing device, a client computing device, and a server computing device. Thereby the same or at least similar advantages and characteristics as with the method of the invention are achieved. Therefore, to avoid repetitions, as regards the following preferred embodiments of the system, the client computing device, and the server computing device, reference is made to above corresponding parts of the description that refer to the method provided by the invention and that discuss the resulting advantages and characteristics.

The object of the invention is also solved by a system for enhancing the efficiency of the use of digital equipment by changing habits of a user of a client computing device. The system comprises a client computing device comprising: a client processor, a client memory, a client transceiver, and a client user interface, wherein the client processor, the client memory, and the client user interface are electronically coupled to each other. The system also comprises a server computing device comprising: a server processor, a server memory, and a server transceiver, wherein the server processor, the server memory, and the server transceiver are electronically coupled to each other. The client computing device, by the client processor executing instructions stored in the client memory and controlling the client transceiver and the client user interface, is configured to: monitor, for a first monitoring time period, the behavior of a user interacting with the client user interface and collecting first user behavior data; and transmit, with the client transceiver, the first user behavior data to the server transceiver. The server computing device, by the server processor executing instructions stored in the server memory and controlling the server transceiver, is configured to: receive, with the server transceiver, the first user behavior data; store, in the server memory, the first user behavior data; determine a user habit, based on the first user behavior data; select a first user habit change stimulus from a plurality of user habit change stimuli, based on the user habit, the first user behavior data, and the behavior data of further users stored in the server memory; transmit, with the server transceiver, the first habit change stimulus to the client transceiver. The client computing device, by the client processor executing instructions stored in the client memory and controlling the client transceiver and the client user interface, is configured to: receive, with the client transceiver, the first user habit change stimulus; apply, via the client user interface, the first user habit change stimulus to the user; monitor, for a second monitoring time period, the behavior of the user interacting with the client user interface and collect second user behavior data; determine whether the user has changed the user habit, based on the second user behavior data; and, if the user has changed the user habit in the desired direction and if there are further user habits to be changed, concentrate on a further user habit.

With some embodiments the client computing device is further configured to: determine, after the first monitoring time, a user baseline, based on the first user behavior data, the user baseline indicating a set of user habits the user actually has and that affect the efficiency of the use of digital equipment; and transmit, with the client transceiver, the user baseline to the server transceiver.

With some embodiments the server computing device determines the user habit, based on the user baseline.

Preferably, the first user habit change stimulus is selected from a plurality of tip stimuli, prime stimuli, nudge stimuli, and feedback stimuli.

With the system provided by the invention a prime stimulus is, for example, a stimulus that influences the near-term future thoughts and actions of a user, even though they may not seem to be connected, a prime stimulus being applied before a user performs a core action to convey a customized message about technology and its features, highlighting its potential for the user.

With the system provided by the invention a nudge stimulus is, for example, a stimulus concerning any aspect of a given choice architecture that alters the behavior of users in a predictable way without removing any options, a nudge stimulus being applied while the user performs an action or an action is taking place, wherein a nudge stimulus includes short sentences or graphical tools to persuade the user towards the desired user behavior.

With the system provided by the invention a feedback stimulus is, for example, helpful information that is given to the user to indicate what can be done to improve adoption after a core action is performed, the feedback stimulus being positive feedback, rewarding the action taken, or corrective feedback, signaling the possibility to correct a previous mistake.

The server computing device of the system provided by the invention is preferably further configured to consider results that have been achieved with other users when it selects the first user habit change stimulus from the plurality of user habit change stimuli.

Also with the system provided by the invention determining the user habit preferably includes determining a core user habit that is needed to enhance the efficiency of the use of digital equipment.

The above mentioned object of the invention is also solved by a client computing device comprising a client processor, a client memory, a client transceiver, and a client user interface. The client processor, the client memory, and the client user interface are electronically coupled to each other; and, for enhancing the efficiency of the use of digital equipment by changing habits of a user of the client computing device, the client computing device, by the client processor executing instructions stored in the client memory and controlling the client transceiver and the client user interface, is configured to: monitor, for a first monitoring time period, the behavior of a user interacting with the client user interface and collecting first user behavior data; transmit, with the client transceiver, the first user behavior data to a server transceiver of a server computing device; receive, with the client transceiver from the server transceiver, a first user habit change stimulus; apply, via the client user interface, the first user habit change stimulus to the user; monitor, for a second monitoring time period, the behavior of the user interacting with the client user interface and collect second user behavior data; determine whether the user has changed the user habit, based on the second user behavior data; and, if the user has changed the user habit in the desired direction and if there are further user habits to be changed, concentrate on a further user habit.

The above mentioned object of the invention is also solved by a server computing device comprising a server processor, a server memory, and a server transceiver. The server processor, the server memory, and the server transceiver are electronically coupled to each other; and, for enhancing the efficiency of the use of digital equipment by changing habits of a user of a client computing device, the server computing device, by the server processor executing instructions stored in the server memory and controlling the server transceiver, is configured to: receive, with the server transceiver from a client transceiver of a client computing device, first user behavior data; store, in the server memory, the first user behavior data; determine a user habit, based on the first user behavior data; select a first user habit change stimulus from a plurality of user habit change stimuli, based on the user habit, the first user behavior data, and the behavior data of further users stored in the server memory; transmit, with the server transceiver to the client transceiver, the first habit change stimulus to the client transceiver; receive, with the server transceiver from the client transceiver, an indication whether the user has changed the user habit in the desired direction, and, if the user has changed the user habit in the desired direction and if there are further user habits to be changed, concentrate on a further user habit, considering second user behavior data received with the server transceiver from the client transceiver.

An important point for the invention is the insight that the adoption of any new behavior encounters physical and psychological resistance. To bring about a new behavior, it is in many cases necessary to "install" a new habit through repeated actions, to create and strengthen new neural connections in the user's brain. To install new habits to empower our professional and personal lives, it has to be understood that, for example, 80% of the effort must be concentrated on this first phase of the process. The science of habits has identified some factors for success: start with small steps that require minimum effort; find a "trigger", an already existing habit, and hook the new one to it; do this every day for at least 30 days; tell someone about it and make sure they're sticking to it; start now! The present invention takes these factors into account.

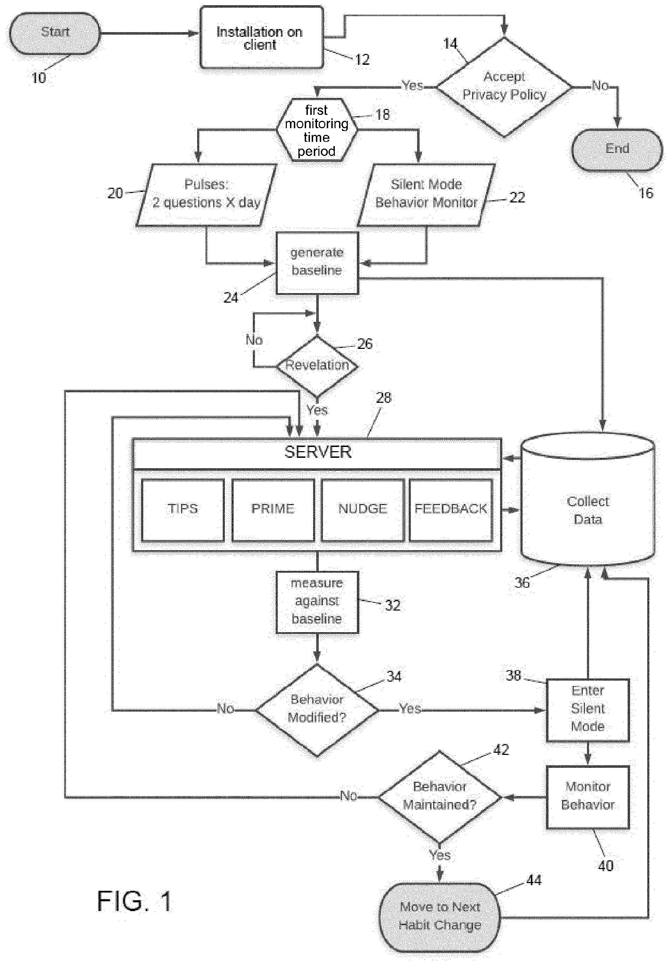

Turning now to FIG. 1 which is a flow chart that illustrates one embodiment of the method in accordance with the invention, and that also illustrates the operation of one embodiment of the system, the client computing device, and the server computing device in accordance with the invention. The method starts at block 10 and the installation of the necessary software on the client computing device takes place at block 12. In block 14 the user is prompted to accept the privacy policy. If the user does not accept the privacy policy, the method ends at block 16. Otherwise a first monitoring time period is started at block 18. In the illustrated example this first monitoring time period can for example last 10 days or less. During the first monitoring time period the client computing device monitors the behavior of the user of the client computing device and collects first user behavior data. It should be clear that, if multiple users use the same client computing device, the user behavior data is collected separately for every user who has accepted the privacy policy. As shown in block 20, during the first monitoring period the software may, for example, ask the user two questions per day to collect meaningful information that can be used for the user behavior data. With some embodiments block 20 can be omitted. In general, during the first monitoring time period the software is in a silent mode, as shown in block 22. Silent mode means that the user does not notice that the software collects the first user behavior data, possibly except when the user is asked any questions in the optional block 20. At the end of first monitoring time period the client computing device determines a user baseline on the basis of the first user behavior data. This user baseline preferably reflects the actual habits the user has (or has not). The user baseline may be a kind of reference that is used later on to check whether the user has improved the behavior. Block 26 is idled until it is time for revelation. Revelation preferably means that silent mode has lapsed and that the software starts to present itself as a digital coach. This digital coach has a given or a selectable personality, and upon revelation it starts to interact with the user.

With the embodiment illustrated in FIG. 1 the first user behavior data and the user baseline are sent to a server computing device. The server computing device selects a suitable first user habit change stimuli from a plurality of tip stimuli, prime stimuli, nudge stimuli, and feedback stimuli that are all stored with or are accessible by the server computing device. The selection is made considering the first user behavior data, the user baseline, and experiences that have been made with other users and that have been stored in block 36. All useful data concerning the user of the client computing device is also stored in block 36 for future use, for example in connection with future users.

After a suitable first user habit change stimulus has been selected at block 28 it is transmitted to the client computing device to be applied to the user via the client user interface that includes, for example, a display, a keyboard, a pointing device, a speaker, a microphone, and so on. After the first user habit change stimulus was applied once or a plurality of times, it is compared with the initial user baseline at block 32 (In practice, preferably, a number of user habit change stimuli are applied on a daily basis, a plurality of times, and the resulting real-time user habit is constantly compared to the initial user baseline, to determine if the desired change in user habit is achieved). If it is than determined in block 34 that the user has not (sufficiently) modified the user habit/behavior, the method returns to block 28 where, for example, a different user habit change stimulus is selected. When it is determined in block 34 that the user has changed the behavior in the desired direction, the method proceeds to block 38 where the silent mode in entered again. The successful application of the first user habit change stimulus is recorded in the database at block 36, and in block 40 the behavior of the user is monitored again, for a second monitoring time period. If it is determined in block 42 that the user has maintained the user habit/behavior in the desired way, the method proceeds to block 44 to concentrate on a further user habit. Otherwise it returns to block 28 to address the first user habit again.

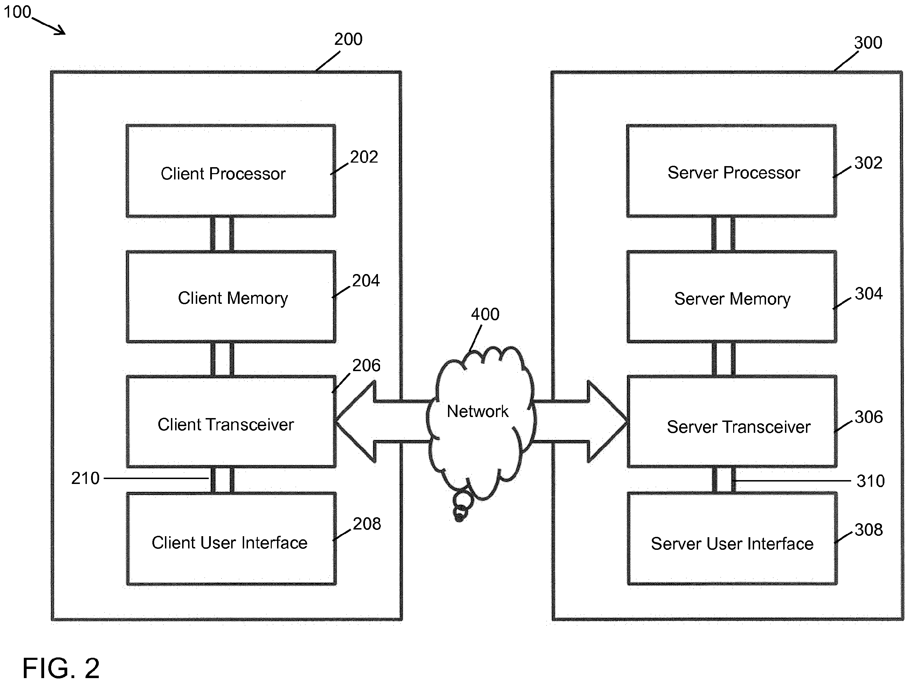

FIG. 2 is block diagram that illustrates one embodiment of the system 100 comprising a client computing device 200 and a server computing device 300 in accordance with the invention, wherein the illustrated system is also capable to execute the method of FIG.1.

The client computing device 200 comprises a client processor 202, a client memory 204, a client transceiver 206, and a client user interface 208. All these components are electronically connected to each other by any suitable conductor means. In the illustrated embodiment the conductor means comprise a client bus 210. The client memory 204 stores data and instructions that can be executed by the client processor 202 to provide the functionality of the present invention. The client transceiver 206 is able to transmit signals to and receive signals from a network 400 in any suitable manner, wired and/or wireless. It is also possible that each transceiver mentioned herein is formed by a separate transmitter and a separate receiver. The client user interface 208 comprises any suitable interface means that enable a user to interact with the client computing device, for example at least one of a screen or touch screen, a keyboard, a pointing device, a microphone, a loudspeaker, and so forth. The client computing device 200 can, for example, be a personal computer, a laptop computer, a tablet computer, a smartphone, a game station, and so forth. Furthermore, the client computing device 200 can be a compact device or any distributed system, as long as it is configured to provide the functionality in accordance with the present invention mentioned above

The server computing device 300 comprises a server processor 302, a server memory 304, a server transceiver 306, and a server user interface 308. All these components are electronically connected to each other by any suitable conductor means. In the illustrated embodiment the conductor means comprise a server bus 310. The server memory 304 stores data and instructions that can be executed by the server processor 302 to provide the functionality of the present invention. The server transceiver 306 is able to transmit signals to and receive signals from a network 400 in any suitable manner, wired and or wireless. The server user interface 308 comprises any suitable interface means that enable a user to interact with the server computing device, for example at least one of a screen or touch screen, a keyboard, a pointing device, a microphone, a loudspeaker, and so forth. The server computing device 300 can, for example, be a traditional server, a personal computer, a laptop computer, a tablet computer, a smartphone, a game station, and so forth. Furthermore, the server computing device 300 can be a compact device or any distributed system, as long as it is configured to provide the functionality in accordance with the present invention mentioned above.

The client transceiver 206 and the server transceiver 306 are capable to exchange data via the network 400 to enable the transmitting and receiving as described above, for example with reference to FIG. 1. The network 400 can be any suitable wired and/or wireless network well known in the art, for example a WAN, a LAN, an intranet, the internet, and so forth, as well as combinations thereof.

While the present invention has been described with respect to a limited number of embodiments, those skilled in the art will appreciate numerous modifications and variations therefrom. It is intended that the appended claims cover all such modifications and variations as fall within the true spirit and scope of this present invention.

Use of the phrase 'configured to,' in one embodiment, refers to arranging, putting together, manufacturing, offering to sell, importing and/or designing an apparatus, hardware, logic, or element to perform a designated or determined task. In this example, an apparatus or element thereof that is not operating is still 'configured to' perform a designated task if it is designed, coupled, and/or interconnected to perform said designated task. As a purely illustrative example, a logic gate may provide a 0 or a 1 during operation. But a logic gate 'configured to' provide an enable signal to a clock does not include every potential logic gate that may provide a 1 or 0. Instead, the logic gate is one coupled in some manner that during operation the 1 or 0 output is to enable the clock. Note once again that use of the term 'configured to' does not require operation, but instead focus on the latent state of an apparatus, hardware, and/or element, where in the latent state the apparatus, hardware, and/or element is designed to perform a particular task when the apparatus, hardware, and/or element is operating.

Instructions used to program logic to perform embodiments of the invention may be stored within a memory in the system, such as DRAM, cache, flash memory, or other storage. Furthermore, the instructions can be distributed via a network or by way of other computer readable media. Thus a machine-readable medium may include any mechanism for storing or transmitting information in a form readable by a machine (e.g., a computer), but is not limited to, floppy diskettes, optical disks, Compact Disc, Read-Only Memory (CD-ROMs), and magneto-optical disks, Read-Only Memory (ROMs), Random Access Memory (RAM), erasable Programmable Read-Only Memory (EPROM), Electrically Erasable Programmable Read-Only Memory (EEPROM), magnetic or optical cards, flash memory, or a tangible, machine-readable storage used in the transmission of information over the Internet via electrical, optical, acoustical or other forms of propagated signals (e.g., carrier waves, infrared signals, digital signals, etc.). Accordingly, the computer-readable medium includes any type of tangible machine-readable medium suitable for storing or transmitting electronic instructions or information in a form readable by a machine (e.g., a computer/computing device).

Moreover, an embodiment can be implemented as a computer-readable storage medium having computer readable code stored thereon for programming a computer (for example, comprising a processor) to perform a method as described and claimed herein. Examples of such computer-readable storage mediums include, but are not limited to, a hard disk, a CD-ROM, an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory) and a Flash memory. Further, it is expected that one of ordinary skill, notwithstanding possibly significant effort and many design choices motivated by, for example, available time, current technology, and economic considerations, when guided by the concepts and principles disclosed herein will be readily capable of generating such software instructions and programs and ICs with minimal experimentation.

### Reference numerals

- 100: System
- 200: Client computing device
- 202: Client processor
- 204: Client memory
- 206: Client transceiver
- 208: Client user interface
- 210: Client bus
- 300: Server computing device
- 302: Server processor
- 304: Server memory
- 306: Server transceiver
- 308: Server user interface
- 310: Server bus
- 400: Network

## Claims

1. A method for enhancing the efficiency of the use of digital equipment by changing habits of a user of a client computing device (200), the method comprising the following steps:
- monitoring, for a first monitoring time period, the behavior of the user interacting with a client user interface (208) of the client computing device (200) and collecting first user behavior data;
- transmitting, with a client transceiver (206) of the client computing device (200), the first user behavior data to a server transceiver (306) of a server computing device (300);
- receiving, with the server transceiver (306), the first user behavior data;
- storing, in a server memory (304) of the server computing device (300), the first user behavior data;
- determining a user habit, based on the first user behavior data;
- selecting, by the server computing device (300), a first user habit change stimulus from a plurality of user habit change stimuli, based on the user habit, the first user behavior data, and behavior data of other users stored in the server memory (304);
- transmitting, with the server transceiver (306), the first habit change stimulus to the client transceiver (206);
- receiving, with the client transceiver (206), the first user habit change stimulus;
- applying, via the client user interface (208), the first user habit change stimulus to the user;
- monitoring, for a second monitoring time period, the behavior of the user interacting with the client user interface (208) and collecting second user behavior data;
- determining whether the user has changed the user habit, based on the second user behavior data; and,
- if the user has changed the user habit in the desired direction and if there are further user habits to be changed, concentrating on a further user habit.

2. The method according to claim 1, further comprising:
- determining, after the first monitoring time period, a user baseline, based on the first user behavior data, the user baseline indicating a set of user habits the user actually has and that affect the efficiency of the use of digital equipment; and
- transmitting, with the client transceiver (206), the user baseline to the server transceiver (306).

3. The method according to claim 2, wherein the step of determining a user habit is performed by the server computing device (300), based on the user baseline.

4. The method according to claim 1, wherein the step of selecting a first user habit change stimulus from the plurality of user habit change stimuli includes selecting from a plurality of tip stimuli, prime stimuli, nudge stimuli, and feedback stimuli.

5. The method according to claim 4, wherein a prime stimulus is a stimulus that influences the near-term future thoughts and actions of a user, even though they may not seem to be connected, a prime stimulus being applied before a user performs a core action to convey a customized message about technology and its features, highlighting its potential for the user.

6. The method according to claim 4, wherein a nudge stimulus is a stimulus concerning any aspect of a given choice architecture that alters the behavior of users in a predictable way without removing any options, a nudge stimulus being applied while the user performs an action or an action is taking place, wherein a nudge stimulus includes short sentences or graphical tools to persuade the user in towards the desired user behavior.

7. The method according to claim 4, wherein a feedback stimulus is helpful information that is given to the user to indicate what can be done to improve adoption after a core action is performed, the feedback stimulus being positive feedback, rewarding the action taken, or corrective feedback, signaling the possibility to correct a previous mistake.

8. The method according to claim 1, wherein the step of selecting a first user habit change stimulus from the plurality of user habit change stimuli includes considering results that have been achieved with other users.

9. The method according to claim 1, wherein the step of determining the user habit includes determining a core user habit that is needed to enhance the efficiency of the use of digital equipment.

10. A system (100) for enhancing the efficiency of the use of digital equipment by changing habits of a user of a client computing device (200), the system comprising:
- the client computing device (200) comprising:
- a client processor (202),
- a client memory (204),
- a client transceiver (206), and
- a client user interface (208),
wherein the client processor (202), the client memory (204), the client transceiver (206) and the client user interface (208) are electronically coupled to each other; and
- a server computing device comprising:
- a server processor (302),
- a server memory (304), and
- a server transceiver (306),
wherein the server processor (302), the server memory (304), and the server transceiver (306) are electronically coupled to each other;
the client computing device (200), by the client processor (202) executing instructions stored in the client memory (204) and controlling the client transceiver (206) and the client user interface (208), being configured to:
- monitor, for a first monitoring time period, the behavior of a user interacting with the client user interface (208) and collecting first user behavior data, and
- transmit, with the client transceiver (206), the first user behavior data to the server transceiver (306);
the server computing device (300), by the server processor (302) executing instructions stored in the server memory (304) and controlling the server transceiver (306), being configured to:
- receive, with the server transceiver (306), the first user behavior data,
- store, in the server memory (304), the first user behavior data,
- determine a user habit, based on the first user behavior data,
- select a first user habit change stimulus from a plurality of user habit change stimuli, based on the user habit, the first user behavior data, and the behavior data of further users stored in the server memory (304),
- transmit, with the server transceiver (306), the first habit change stimulus to the client transceiver (206);
the client computing device (200), by the client processor (202) executing instructions stored in the client memory (204) and controlling the client transceiver (206) and the client user interface (208), being configured to:
- receive, with the client transceiver (206), the first user habit change stimulus,
- apply, via the client user interface (208), the first user habit change stimulus to the user,
- monitor, for a second monitoring time period, the behavior of the user interacting with the client user interface (208) and collect second user behavior data,
- determine whether the user has changed the user habit, based on the second user behavior data, and,
- if the user has changed the user habit in the desired direction and if there are further user habits to be changed, concentrate on a further user habit.

11. The system (100) according to claim 10, wherein the client computing device (200) is further configured to:
- determine, after the first monitoring time period, a user baseline, based on the first user behavior data, the user baseline indicating a set of user habits the user actually has and that affect the efficiency of the use of digital equipment; and
- transmit, with the client transceiver (206), the user baseline to the server transceiver (306).

12. The system (100) according to claim 11, wherein the server computing device (300) determines the user habit, based on the user baseline.

13. The system (100) according to claim 10, wherein the first user habit change stimulus is selected from a plurality of tip stimuli, prime stimuli, nudge stimuli, and feedback stimuli.

14. The system (100) according to claim 13, wherein a prime stimulus is a stimulus that influences the near-term future thoughts and actions of a user, even though they may not seem to be connected, a prime stimulus being applied before a user performs a core action to convey a customized message about technology and its features, highlighting its potential for the user.

15. The system (100) according to claim 13, wherein a nudge stimulus is a stimulus concerning any aspect of a given choice architecture that alters the behavior of users in a predictable way without removing any options, a nudge stimulus being applied while the user performs an action or an action is taking place, wherein a nudge stimulus includes short sentences or graphical tools to persuade the user in towards the desired user behavior.

16. The system (100) according to claim 13, wherein a feedback stimulus is helpful information that is given to the user to indicate what can be done to improve adoption after a core action is performed, the feedback stimulus being positive feedback, rewarding the action taken, or corrective feedback, signaling the possibility to correct a previous mistake.

17. The system (100) according to claim 10, wherein the server computing device (300) is further configured to consider results that have been achieved with other users when it selects the first user habit change stimulus from the plurality of user habit change stimuli.

18. The system (100) according to claim 10, wherein determining the user habit includes determining a core user habit that is needed to enhance the efficiency of the use of digital equipment.

19. A client computing device (200) comprising:
- a client processor (202);
- a client memory (204);
- a client transceiver (206); and
- a client user interface (208);
wherein the client processor (202), the client memory (204), and the client user interface (208) are electronically coupled to each other; and wherein, for enhancing the efficiency of the use of digital equipment by changing habits of a user of the client computing device (200), the client computing device (200), by the client processor (202) executing instructions stored in the client memory (204) and controlling the client transceiver (206) and the client user interface (208), is configured to:
- monitor, for a first monitoring time period, the behavior of a user interacting with the client user interface (208) and collecting first user behavior data; and
- transmit, with the client transceiver (206), the first user behavior data to a server transceiver (306) of a server computing device (300);
- receive, with the client transceiver (206) from the server transceiver (306), a first user habit change stimulus;
- apply, via the client user interface (208), the first user habit change stimulus to the user;
- monitor, for a second monitoring time period, the behavior of the user interacting with the client user interface (208) and collect second user behavior data;
- determine whether the user has changed the user habit, based on the second user behavior data; and,
- if the user has changed the user habit in the desired direction and if there are further user habits to be changed, concentrate on a further user habit.

20. A server computing device (300) comprising:
- a server processor (302);
- a server memory (304); and
- a server transceiver (306);
wherein the server processor (302), the server memory (304), and the server transceiver (306) are electronically coupled to each other; and wherein, for enhancing the efficiency of the use of digital equipment by changing habits of a user of a client computing device (200), the server computing device (300), by the server processor (302) executing instructions stored in the server memory (304) and controlling the server transceiver (306), being configured to:
- receive, with the server transceiver (306) from a client transceiver (206) of a client computing device (200), first user behavior data,
- store, in the server memory (304), the first user behavior data;
- determine a user habit, based on the first user behavior data;
- select a first user habit change stimulus from a plurality of user habit change stimuli, based on the user habit, the first user behavior data, and the behavior data of further users stored in the server memory (304);
- transmit, with the server transceiver (306) to the client transceiver (206), the first habit change stimulus to the client transceiver;
- receive, with the server transceiver (306) from the client transceiver (206), an indication whether the user has changed the user habit in the desired direction, and,
- if the user has changed the user habit in the desired direction and if there are further user habits to be changed, concentrate on a further user habit, considering second user behavior data received with the server transceiver (306) from the client transceiver (206).
